# EUROPEAN PATENT APPLICATION

(11) **EP 4 445 892 A1**
(43) Date of publication of application: **16.10.2024**
(21) Application number: 24152772.0
(22) Date of filing: 19.01.2024
(51) Int. Cl.: A61K 8/04, A61K 36/346, A61K 36/42, A61K 36/484, A61K 36/53, A61K 36/539, A61K 36/68, A61K 36/718, A61K 36/725, A61K 36/73, A61K 36/752, A61K 36/8968, A61K 36/9068, A61P 1/02, A61P 1/04, A61Q 11/00

(54) **MODIFIED PRESCRIPTION OF GANCAO XIEXIN DECOCTION AND USE THEREOF**

(30) Priority: 10.04.2023 CN 202310375975
(71) Applicant: Infinitus (China) Company Ltd., Jiangmen, Guangdong 529156 (CN)
(72) Inventor: Xiao, Lei, Jiangmen, Guangdong, 529156 (CN); He, Lingling, Jiangmen, Guangdong, 529156 (CN); Huang, Jinlian, Jiangmen, Guangdong, 529156 (CN); Gao, Yan, Jiangmen, Guangdong, 529156 (CN)
(74) Representative: Petraz, Gilberto Luigi

(57) **Abstract**

Disclosed in the present invention is a modified prescription of Gancao Xiexin decoction and the use thereof. The modified prescription of Gancao Xiexin decoction of the present invention mainly contains *Coptidis Rhizoma, Scutellariae Radix, Glycyrrhizae Radix et Rhizoma, Zingiberis Rhizoma, Jujubαe Fructus, Citri Reticulatae Pericarpium* and *Bolbostemmatis Rhizomα* with effects of alleviating inflammatory reaction and promoting oral healing, and can be used for treating oral ulcer or for preparing a product for treating oral ulcer, which helps reduce the recurrence of oral ulcer. Meanwhile, the present invention also provides a soft gel containing the aqueous extract of the modified prescription of Gancao Xiexin decoction, which has a simple composition, does not contain toxic traditional Chinese medicine, can be administered by coating oral ulcer, is convenient to use and has a good therapeutic effect. The present invention not only enriches the traditional Chinese medicine composition for treating oral ulcer but also has important significance for the development and application of Gancao Xiexin decoction.

## Description

### Technical Field

The present invention belongs to the technical field of traditional Chinese medicine. More particularly, it relates to a modified prescription of Gancao Xiexin decoction and use thereof.

### Background Art

The oral ulcer is a common ulcerative injury of the human oral mucosa, which can occur in any part of the oral cavity, but it is more common in the inside of the lip, tip of the tongue, abdomen of the tongue, buccal mucosa or gingiva. Oral ulcer is self-limited, but because of their easy recurrence, it often causes severe pain, and seriously affects eating and speaking, causing great trouble to patients. It is believed in western medicine that oral ulcers are due to body induced by genetic factors, infection factors, systemic diseases, hyperoxia free radical attack, psychological factors or lack of nutritional elements, and other reasons, there is an imbalance among the body's immune response, external stimulation, damaged parts repair, resulting in mucosal ulcer, gingival tissue destruction, periodontal repair difficulties and other symptoms. Western medicine treatment of oral ulcers is also mostly anti-inflammatory, analgesic, secondary infection prevention, and healing promotion as the principle. For example, use of patches containing dexamethasone acetate or metronidazole, oral vitamins, cydiodine tablets, lysozyme buccal tablets, etc.; or use gargle containing metronidazole, chlorhexidine, benzydamine or diclofenac, apply dyclonine or procaine and lidocaine gel to relieve pain. For severe oral mucositis, palifermin (KGF-1, recombinant human keratinocyte growth factor -1) can also be injected; or use of local physical therapy including laser, low-frequency ultrasound, chemical corrosion (e.g. silver nitrate), physical barriers (e.g. Cyanoacrylate adhesive), etc. However, the above treatments are limited to anti-inflammatory, analgesic, and healing promotion in the affected area, without an overall regulation of the patient's constitution.

According to traditional Chinese medicine, oral ulcer belongs to the category of "chyme" and "aphtha", and their causes and pathogenesis include emotional imbalance, stagnation of qi circulation, dietary irregularities, and excessive stomach heat due to spleen and stomach disharmony. It primarily involves the organs of the heart, spleen, and stomach. In the process of treating oral ulcers, Traditional Chinese Medicine (TCM) aims to alleviate the clinical symptoms of patients while also regulating their constitution through the elimination of excessive heart fire and the clearing of heat toxins, achieving a dual effect of treating both the root cause and the symptoms.

Gancao Xiexin decoction (licorice decoction to drain the epigastrium), originating from *Synopsis of the Golden Chamber* and *Treatise on Febrile Diseases,* is mainly composed of *Glycyrrhizae Radix et Rhizoma, Scutellariae Radix, Coptidis Rhizoma, Codonopsis Radix, Pinelliae Rhizoma, Zingiberis Rhizoma,* and *Jujubαe Fructus.* In the *Synopsis of the Golden Chamber,* it is primarily used for treating infectious parasites, damp-heat stagnation leading to delirium; while in the *Treatise on Febrile Diseases,* it is commonly used for treating recurrent diarrhea, weak spleen and stomach, and alternating cold and heat symptoms. Currently, it is also used for treating oral ulcers. However, the whole plant of *Pinelliae Rhizoma* in Gancao Xiexin decoction contains toxins, with the tuber having a higher toxicity. Ingesting 0.1-1.8 g of the raw plant can cause poisoning, and symptoms can appear within 0.5-2 h. Ingesting a small amount can result in numbness of the mouth and tongue while ingesting a larger amount can cause burning pain, swelling, inability to speak, drooling, vomiting, numbness of the whole body, slow and irregular breathing, spasms, and difficulty breathing. Eventually, death can occur due to paralysis of the respiratory center. In order to reduce the toxicity of raw *Pinelliae Rhizoma,* processed *Pinelliae Rhizoma,* such as *Pinelliae Rhizoma* Praeparatum Cum Zingibere et Alumine, and *Pinelliae Rhizoina* Preparatum, etc. will be used in common prescription. Although the contents of toxic components such as calcium oxalate needle crystals, lectin proteins, and alkaloids in raw *Pinelliae Rhizoma* are significantly decreased after processing, the effective components such as ephedrine, protein, total sugar, organic acid, inosine, guanosine, adenosine, succinic acid, ephedrine hydrochloride, and β-sitosterol are also decreased. In addition, *Pinelliae Rhizoma* has not been on the list of used cosmetics, which also limits the use of Gancao Xiexin decoction. Therefore, it is of great significance for the development and use of Gancao Xiexin decoction to develop the modified prescription of Gancao Xiexin decoction with better therapeutic effect than the original prescription.

### Summary of the Invention

The technical problem to be solved by the present invention is to overcome the defects and deficiencies of the prior art mentioned above and provide a modified prescription of Gancao Xiexin decoction and the use thereof.

It is the first object of the present invention to provide a modified prescription of Gancao Xiexin decoction.

A second object of the present invention is to provide the use of the modified prescription of Gancao Xiexin decoction or the extract thereof in the preparation of a product for the treatment of oral ulcers. A third object of the present invention is to provide a product for treating oral ulcers.

A fourth object of the present invention is to provide a soft gel for treating oral ulcers.

A fifth object of the present invention is to provide a method for preparing the soft gel.

The above object of the present invention is achieved by the following technical solution:
The present invention provides a modified prescription of Gancao Xiexin decoction, the modified prescription includes, in parts by weight, 2-5 parts of *Coptidis Rhizoma,* 6-12 parts of *Scutellariae Radix,* 10-15 parts of *Glycyrrhizae Radix et Rhizoma,* 6-12 parts of *Zingiberis Rhizoma,* 5-12 parts of *Jujubαe Fructus,* 10-15 parts of *Citri Reticulatae Pericarpium,* and 10-15 parts of *Bolbostemmatis Rhizoma.*

Preferably, the modified prescription includes, in parts by weight, 2-3 parts of *Coptidis Rhizoma,* 9-12 parts of *Scutellariae Radix,* 10-12 parts of *Glycyrrhizae Radix et Rhizoma,* 6-9 parts of *Zingiberis Rhizoma,* 9-12 parts of *Jujubαe Fructus,* 10-12 parts of *Citri Reticulatae Pericarpium,* and 12-15 parts of *Bolbostemmatis Rhizoma.*

Preferably, the modified prescription includes, in parts by weight, 3 parts of *Coptidis Rhizoma, 9* parts of *Scutellariae Radix,* 12 parts of *Glycyrrhizae Radix et Rhizoma,* 9 parts of *Zingiberis Rhizoma,* 9 parts of *Jujubαe Fructus,* 12 parts of *Citri Reticulatae Pericarpium,* and 12 parts of *Bolbostemmatis Rhizoma.*

The present invention further provides a modified prescription of Gancao Xiexin decoction, the modified prescription includes, in parts by weight, 2-5 parts of *Coptidis Rhizoma,* 6-12 parts of *Scutellariae Radix,* 10-15 parts of *Glycyrrhizae Radix et Rhizoma,* 6-12 parts of *Zingiberis Rhizoma,* 5-12 parts of *Jujubαe Fructus,* 10-15 parts of *Citri Reticulatae Pericarpium,* and 10-15 parts of *Bolbostemmatis Rhizoma,* as well as 1-5 parts of *Pogostemonis Herba* and 1-5 parts of *Eupatorii Herba.*

Preferably, the modified prescription includes, in parts by weight, 2-3 parts of *Coptidis Rhizoma,* 9-12 parts of *Scutellariae Radix,* 10-12 parts of *Glycyrrhizae Radix et Rhizoma,* 6-9 parts of *Zingiberis Rhizoma,* 9-12 parts of *Jujubαe Fructus,* 10-12 parts of *Citri Reticulatae Pericarpium,* and 12-15 parts of *Bolbostemmatis Rhizoma,* as well as 3-5 parts of *Pogostemonis Herba* and 3-5 parts of *Eupatorii Herba.*

Preferably, the modified prescription includes, in parts by weight, 3 parts of *Coptidis Rhizoma, 9* parts of *Scutellariae Radix,* 12 parts of *Glycyrrhizae Radix et Rhizoma,* 9 parts of *Zingiberis Rhizoma,* 9 parts of *Jujubαe Fructus,* 12 parts of *Citri Reticulatae Pericarpium,* and 12 parts of *Bolbostemmatis Rhizoma,* as well as 3 parts of *Pogostemonis Herba* and 3 parts of *Eupatorii Herba.* The present invention further provides a modified prescription of Gancao Xiexin decoction, the modified prescription includes, in parts by weight, 2-5 parts of *Coptidis Rhizoma,* 6-12 parts of *Scutellariae Radix,* 10-15 parts of *Glycyrrhizae Radix et Rhizoma,* 6-12 parts of *Zingiberis Rhizoma,* 5-12 parts of *Jujubαe Fructus,* 10-15 parts of *Citri Reticulatae Pericarpium,*10-15 parts of *Bolbostemmatis Rhizoma,* and 1-8 parts of *Plαtycodonis Radix.*

Preferably, the modified prescription includes, in parts by weight, 2-3 parts of *Coptidis Rhizoma,* 9-12 parts of *Scutellariae Radix,* 10-12 parts of *Glycyrrhizae Radix et Rhizoma,* 6-9 parts of *Zingiberis Rhizoma,* 9-12 parts of *Jujubαe Fructus,* 10-12 parts of *Citri Reticulatae Pericarpium,* 12-15 parts of *Bolbostemmatis Rhizoma,* and 6-8 parts of *Platycodonis Radixs.*

Preferably, the modified prescription includes, in parts by weight, 3 parts of *Coptidis Rhizoma, 9* parts of *Scutellariae Radix,* 12 parts of *Glycyrrhizae Radix et Rhizoma,* 9 parts of *Zingiberis Rhizoma,* 9 parts of *Jujubαe Fructus,* 12 parts of *Citri Reticulatae Pericarpium,* 12 parts of *Bolbostemmatis Rhizoma,* and 6 parts of *Platycodonis Radix.*

The present invention further provides a modified prescription of Gancao Xiexin decoction, the modified prescription includes, in parts by weight, 2-5 parts of *Coptidis Rhizoma,* 6-12 parts of *Scutellariae Radix,* 10-15 parts of *Glycyrrhizae Radix et Rhizoma,* 6-12 parts of *Zingiberis Rhizoma,* 5-12 parts of *Jujubαe Fructus,* 10-15 parts of *Citri Reticulatae Pericarpium,*10-15 parts of *Bolbostemmatis Rhizoma,* 1-8 parts of *Platycodonis Radix,* 10-20 parts of *Scrophulariae Radix,* and 10-20 parts of *Ophiopogonis Radix.*

Preferably, the modified prescription includes, in parts by weight, 2-3 parts of *Coptidis Rhizoma,* 9-12 parts of *Scutellariae Radix,* 10-12 parts of *Glycyrrhizae Radix et Rhizoma,* 6-9 parts of *Zingiberis Rhizoma,* 9-12 parts of *Jujubαe Fructus,* 10-12 parts of *Citri Reticulatae Pericarpium,* 12-15 parts of *Bolbostemmatis Rhizoma,* 1-6 parts of *Platycodonis Radix,* 10-18 parts of *Scrophulariae Radix,* and 10-18 parts of *Ophiopogonis Radix.*

More preferably, the modified prescription includes, in parts by weight, 3 parts of *Coptidis Rhizoma,* 9 parts of *Scutellariae Radix,* 12 parts of *Glycyrrhizae Radix et Rhizoma,* 9 parts of *Zingiberis Rhizoma,* 9 parts of *Jujubαe Fructus,* 12 parts of *Citri Reticulatae Pericarpium,* 12 parts of *Bolbostemmatis Rhizoma,* 6 parts of *Platycodonis Radix,* 18 parts of *Scrophulariae Radix,* and 18 parts of *Ophiopogonis Radix.*

It was found in the experiments of the present invention that the soft gel prepared from the above-mentioned modified prescription of Gancao Xiexin decoction has a certain improvement effect on oral ulcers. Therefore, the present invention claims the use of any one of the above-mentioned modified prescriptions of Gancao Xiexin decoction or the extract thereof in the preparation of a product for treating oral ulcers.

The present invention also provides a product for treating oral ulcers including any of the above-described modified prescriptions of Gancao Xiexin decoction or an extract thereof. Specifically, the extract is an aqueous extract of the modified prescription of Gancao Xiexin decoction.

The preparation of the aqueous extract of the modified prescription of Gancao Xiexin decoction includes the following steps:
S1, taking medicinal materials according to the modified prescription, removing insects or metamorphic parts thereof, crushing the medicinal materials and sieving to obtain crude medicinal powder;
S2, adding pure water to the crude medicinal powder obtained in step S1 in a ratio of solid to liquid of 1: (10-40), heating, refluxing to extract and obtaining an extract liquid; and S3, centrifuging the extract liquid obtained in step S2, and concentrating a supernatant in vacuo to obtain the aqueous extract of the modified prescription of Gancao Xiexin decoction, and storing the same at 4°C.

Specifically, the sieving of step S1 is performed with a 60-mesh screen.

Specifically, the heating and refluxing to extract of step S2 is as follows: after adding water to the medicinal material powder, boiling, and refluxing to extract at 100°C for 0.5 h, and pouring out the liquid to be retained; adding pure water to the medicinal residue, boiling and refluxing for extraction for another 0.5 h, pouring out the liquid and combining with the previous liquid to obtain the extract liquid.

Specifically, centrifugation in step S3 is performed under the conditions as follows: centrifuging at 4000 r/min for 15 min.

The present invention also provides a soft gel for treating oral ulcers including an aqueous extract of any of the above-described modified prescriptions of Gancao Xiexin decoction.

The method for preparing the soft gel for treating oral ulcers is: diluting the aqueous extract of the modified prescription of Gancao Xiexin decoction with water, adding sodium carboxymethyl cellulose, glycerol, and sucralose for heating and dissolving, and cooling to obtain the soft gel. Specifically, the aqueous extract of the modified prescription of Gancao Xiexin decoction is diluted to a constant volume of 10 mL with water, 4 g of sodium carboxymethyl cellulose, 20 g of glycerol, and 0.015 g of sucralose are added, followed by being heated to dissolve and then cooled to obtain the soft gel.

The present invention has the following advantageous effects:
The present invention provides a modified prescription of Gancao Xiexin decoction with the effect of promoting the healing of oral ulcers, which is simple in composition, does not contain toxic traditional Chinese medicine, and can be used for preparing a product for treating oral ulcer. On the basis of the modified prescription, the present invention also provides a soft gel including an aqueous extract of the modified prescription, which can be administered by coating an oral ulcer, and which is easy to use and effective. The present invention enriches the traditional Chinese medicine composition that can be used to treat oral ulcers and also has important significance for the development and application of Gancao Xiexin decoction.

### Brief Description of the Drawings

FIG. 1 is images showing the healing process of a typical oral ulcer in mice.
FIG. 2 is a heat map of mice's oral ulcer score.
FIG. 3 is a curve of average body weight change of mice.
FIG. 4 shows the statistical results of the significance of differences in average body weight change of mice.
FIG. 5 shows the results of changes in the content of IL-6 in the blood of mice.
FIG. 6 shows the results of changes in the content of IL-1α in the blood of mice.
FIG. 7 shows the results of changes in the content of IL-10 in the blood of mice.
FIG. 8 shows the results of changes in the content of IL-35 in the blood of mice.

### Detailed Description of the Invention

The invention will now be further described with reference to the accompanying drawings and specific examples, which are not intended to limit the invention in any way. Unless otherwise indicated, the reagents, methods, and equipment used herein are those conventional in the art. Unless otherwise noted, the reagents and materials used in the following examples are commercially available.

The modified prescription of Gancao Xiexin decoction described in the examples of the present invention is all in one dose amount.

### Example 1 Preparation of aqueous extract of modified prescription of Gancao Xiexin decoction

The modified prescription of Gancao Xiexin decoction described in this example is composed of *Coptidis Rhizoma, Scutellariae Radix, Glycyrrhizae Radix et Rhizoma, Zingiberis Rhizoma, Jujubαe Fructus, Citri Reticulatae Pericarpium,* and *Bolbostemmatis Rhizoma,* and is denoted as modified prescription A of Gancao Xiexin decoction.

In this example, a total of three aqueous extracts of modified prescription of Gancao Xiexin decoction with different parts by weight of each Chinese medicine were prepared, and the composition of each composition is specifically as follows:
*(1) Coptidis Rhizoma* 3 g, *Scutellariae Radix* 9 g, *Glycyrrhizae Radix et Rhizoma* 12 g, *Zingiberis Rhizoma* 9 g, *Jujubαe Fructus* 9 g, *Citri Reticulatae Pericarpium* 12 g, *Bolbostemmatis Rhizoma 12* g;
*(2) Coptidis Rhizoma* 2 g, *Scutellariae Radix* 6 g, *Glycyrrhizae Radix et Rhizoma* 10 g, *Zingiberis Rhizoma* 6 g, *Jujubαe Fructus* 5 g, *Citri Reticulatae Pericarpium* 10 g, *Bolbostemmatis Rhizoma* 10 g;
*(3) Coptidis Rhizoma* 5 g, *Scutellariae Radix* 12 g, *Glycyrrhizae Radix et Rhizoma* 15 g, *Zingiberis Rhizoma* 12 g, *Jujubαe Fructus* 12 g, *Citri Reticulatae Pericarpium* 15 g, *Bolbostemmatis Rhizoma* 15 g.

The method for preparing the aqueous extract of the modified prescription of Gancao Xiexin decoction is as follows:
S1, taking medicinal materials according to the modified prescription, removing insects or metamorphic parts thereof, grinding the medicinal materials with a powder grinder, and screening through a 60-mesh sieve to obtain medicinal material powder;
S2, adding pure water to the crude medicinal powder obtained in step S1 in a ratio of solid to liquid of 1:10, boiling and refluxing at 100°C to extract for 0.5 hours, and pouring out the liquid to be retained; adding pure water to the medicinal residue in a ratio of solid to liquid of 1:10, boiling and refluxing for extraction for another 0.5 h, pouring out the liquid and combining with the previous liquid to obtain an extract liquid; and
S3, centrifuging the extract liquid obtained in step S2 at 4000 r/min for 15 min, taking the supernatant, and concentrating the supernatant in vacuo to a concentration of about 0.18 g/mL to obtain the aqueous extract of the modified prescription of Gancao Xiexin decoction, and storing in a refrigerator at 4°C for later use.

### Example 2 Preparation of aqueous extract of modified prescription of Gancao Xiexin decoction

The modified prescription of Gancao Xiexin decoction described in this example is composed of *Coptidis Rhizoma, Scutellariae Radix, Glycyrrhizae Radix et Rhizoma, Zingiberis Rhizoma, Jujubαe Fructus, Citri Reticulatae Pericarpium, Bolbostemmatis Rhizoma, Pogostemonis Herba,* and *Eupatorii Herba,* and is denoted as modified prescription B of Gancao Xiexin decoction.

In this example, a total of three aqueous extracts of modified prescription of Gancao Xiexin decoction with different parts by weight of each Chinese medicine were prepared, and the composition of each composition is specifically as follows:
*(1) Coptidis Rhizoma* 3 g, *Scutellariae Radix* 9 g, *Glycyrrhizae Radix et Rhizoma* 12 g, *Zingiberis Rhizoma* 9 g, *Jujubαe Fructus* 9 g, *Citri Reticulatae Pericarpium* 12 g, *Bolbostemmatis Rhizoma 12* g, *Pogostemonis Herba* 3 g, *Eupatorii Herba* 3 g;
*(2) Coptidis Rhizoma* 2 g, *Scutellariae Radix* 6 g, *Glycyrrhizae Radix et Rhizoma* 10 g, *Zingiberis Rhizoma* 6 g, *Jujubαe Fructus* 5 g, *Citri Reticulatae Pericarpium* 10 g, *Bolbostemmatis Rhizoma* 10 g, *Pogostemonis Herba* 1 g, *Eupatorii Herba* 1 g;
*(3) Coptidis Rhizoma* 5 g, *Scutellariae Radix* 12 g, *Glycyrrhizae Radix et Rhizoma* 15 g, *Zingiberis Rhizoma* 12 g, *Jujubαe Fructus* 12 g, *Citri Reticulatae Pericarpium* 15 g, *Bolbostemmatis Rhizoma* 15 g, *Pogostemonis Herba* 5 g, *Eupatorii Herba* 5 g.

The aqueous extract of the modified prescription of Gancao Xiexin decoction was prepared in the same manner as in Example 1.

### Example 3 Preparation of aqueous extract of modified prescription of Gancao Xiexin decoction

The modified prescription of Gancao Xiexin decoction described in this example is composed of *Coptidis Rhizoma, Scutellariae Radix, Glycyrrhizae Radix et Rhizoma, Zingiberis Rhizoma, Jujubαe Fructus, Citri Reticulatae Pericarpium, Bolbostemmatis Rhizoma,* and *Platycodonis Radix,* and is denoted as modified prescription C of Gancao Xiexin decoction.

In this example, a total of three aqueous extracts of modified prescription of Gancao Xiexin decoction with different parts by weight of each Chinese medicine were prepared, which is specifically as follows:
*(1) Coptidis Rhizoma* 3 g, *Scutellariae Radix* 9 g, *Glycyrrhizae Radix et Rhizoma* 12 g, *Zingiberis Rhizoma* 9 g, *Jujubαe Fructus* 9 g, *Citri Reticulatae Pericarpium* 12 g, *Bolbostemmatis Rhizoma 12* g, *Platycodonis Radix* 6 g;
*(2) Coptidis Rhizoma* 2 g, *Scutellariae Radix* 6 g, *Glycyrrhizae Radix et Rhizoma* 10 g, *Zingiberis Rhizoma* 6 g, *Jujubαe Fructus* 5 g, *Citri Reticulatae Pericarpium* 10 g, *Bolbostemmatis Rhizoma* 10 g, *Platycodonis Radix* 1g;
*(3) Coptidis Rhizoma* 5 g, *Scutellariae Radix* 12 g, *Glycyrrhizae Radix et Rhizoma* 15 g, *Zingiberis Rhizoma* 12 g, *Jujubαe Fructus* 12 g, *Citri Reticulatae Pericarpium* 15 g, *Bolbostemmatis Rhizoma* 10 g, *Platycodonis Radix* 6 g.

The aqueous extract of the modified prescription of Gancao Xiexin decoction was prepared in the same manner as in Example 1.

### Example 4 Preparation of aqueous extract of modified prescription of Gancao Xiexin decoction

The modified prescription of Gancao Xiexin decoction described in this example is composed of *Coptidis Rhizoma, Scutellariae Radix, Glycyrrhizae Radix et Rhizoma, Zingiberis Rhizoma, Jujubαe Fructus, Citri Reticulatae Pericarpium, Bolbostemmatis Rhizoma, Platycodonis Radix, Scrophulariae Radix,* and *Ophiopogonis Radix,* and is denoted as modified prescription D of Gancao Xiexin decoction.

In this example, a total of three aqueous extracts of modified prescription of Gancao Xiexin decoction with different parts by weight of each Chinese medicine were prepared, which is specifically as follows:
*(1) Coptidis Rhizoma* 3 g, *Scutellariae Radix* 9 g, *Glycyrrhizae Radix et Rhizoma* 12 g, *Zingiberis Rhizoma* 9 g, *Jujubαe Fructus* 9 g, *Citri Reticulatae Pericarpium* 12 g, *Bolbostemmatis Rhizoma 12* g, *Platycodonis Radix* 6 g, *Scrophulariae Radix* 18 g, *Ophiopogonis Radix* 18 g;
*(2) Coptidis Rhizoma* 2 g, *Scutellariae Radix* 6 g, *Glycyrrhizae Radix et Rhizoma* 10 g, *Zingiberis Rhizoma* 6 g, *Jujubαe Fructus* 5 g, *Citri Reticulatae Pericarpium* 10 g, *Bolbostemmatis Rhizoma* 10 g, *Platycodonis Radix* 1 g, *Scrophulariae Radix* 10 g, *Ophiopogonis Radix* 10 g;
*(3) Coptidis Rhizoma* 5 g, *Scutellariae Radix* 12 g, *Glycyrrhizae Radix et Rhizoma* 15 g, *Zingiberis Rhizoma* 12 g, *Jujubαe Fructus* 12 g, *Citri Reticulatae Pericarpium* 15 g, *Bolbostemmatis Rhizoma* 15 g, *Platycodonis Radix* 6 g, *Scrophulariae Radix* 20 g, *Ophiopogonis Radix* 20 g.

The aqueous extract of the modified prescription of Gancao Xiexin decoction was prepared in the same manner as in Example 1.

### Effect Example 1

In order to test the effects of the modified prescription of Gancao Xiexin decoction described in Examples 1-4 on treating oral ulcers, the present invention prepared soft gels using the aqueous extracts of the modified prescription of Gancao Xiexin decoction prepared in Examples 1-4, respectively.

### 1. Preparation of the soft gel

The method for preparing the soft gel is as follows: the aqueous extract of the modified prescription of Gancao Xiexin decoction was diluted to a constant volume of 10 mL with water, 4 g of sodium carboxymethyl cellulose, 20 g of glycerol and 0.015 g of sucralose were added, heated to dissolve and then cooled to obtain the soft gel.

As a negative control, a soft gel without a drug was prepared according to the present invention, and the aqueous extract solution was replaced with PBS solution and prepared in the same manner.

As a positive control, soft gels containing 0.75 mg/mL of recombinant human keratinocyte growth factor (KGF-1) and 0.375 mg/mL of recombinant human keratinocyte growth factor respectively, were prepared according to the present invention, i.e. the aqueous extract was replaced with recombinant human keratinocyte growth factor and prepared in the same manner.

Meanwhile, according to the present invention, an aqueous extract of an original prescription of Gancao Xiexin decoction was prepared by the method described in Example 1, and a soft gel was prepared by the same method. The original prescription of Gancao Xiexin decoction is composed of *Coptidis Rhizoma* 3 g, *Scutellariae Radix* 9 g, *Glycyrrhizae Radix et Rhizoma* 12 g, *Zingiberis Rhizoma* 9 g, *Jujubαe Fructus* 9 g, *Pinelliae Rhizoma* 12 g.

### 2. Animal experiments

### (1) Experimental animal

Female Kunming mice, purchased from Hubei Experimental Animal Research Center, aged 4-6 weeks, 22-25 g, were bred in ABSL-2 laboratory at room temperature of 20 ± 2°C, with natural relative humidity, free drinking water, natural light, and bred for 1-2 weeks. The experiments were conducted after the mice showed no abnormality in activities and signs.

### (2) Modeling of oral ulcer and administration

The mice were divided into blank control group, negative control group, positive control group (KGF-1 and original prescription of Gancao Xiexin decoction") and experimental group (modified prescription A, B, C, and D of Gancao Xiexin decoction), in which the positive control group and experimental group were divided into high-dose group and low-dose group, with 12 mice in each group.

Dosing was determined as follows:
The common dose of Gancao Xiexin decoction for adults is 1 dose/day. The standard body weight of adults is estimated as 60 kg. The daily standard body weight dose of adults = 1/60 ≈ 0.017 dose/kg body weight. According to the method described in *Methodology for Pharmacological Study of Traditional Chinese Medicine,* the conversion factor of drug administration from adult to mouse is 9.1. According to this factor, the standard daily body weight dose of a mouse = 0.017 × 9.1 ≈ 0.1547 dose/kg body weight. The daily standard body weight dose of mice was taken as the low sample dose; The sample dose for the high dose was calculated as twice the dose for the low sample and was 0.3094 dose/kg body weight. Each mouse was weighed at 25 g and converted to a daily dose per mouse as shown in Table 1 below. According to the concentration and volume of the extract liquid of each prescription, prepare the soft gel by converting the sample volume of the crude drug in mice, and administer 100 µL of soft gel containing a sample to each mouse every day.

**Table 1 Daily dose of mice**

| Gancao Xiexin decoction | Sample dose for mice (g crude drug/animal) | |
|---|---|---|
| | Low dose | High dose |
| Original prescription | 0.2 | 0.4 |
| Modified prescription A (1) | 0.25 | 0.5 |
| Modified prescription A (2) | 0.2 | 0.4 |
| Modified prescription A (3) | 0.33 | 0.66 |
| Modified prescription B (1) | 0.275 | 0.55 |
| Modified prescription B (2) | 0.197 | 0.394 |
| Modified prescription B (3) | 0.371 | 0.742 |
| Modified prescription C (1) | 0.275 | 0.55 |
| Modified prescription C (2) | 0.193 | 0.386 |
| Modified prescription C (3) | 0.336 | 0.672 |
| Modified prescription D (1) | 0.4 | 0.8 |
| Modified prescription D (2) | 0.27 | 0.54 |
| Modified prescription D (3) | 0.51 | 1.02 |

Molding: mice were maintained in tetracycline-containing drinking water (2.5 g/L) for 5 days and were immunosuppressed by intramuscular injection of 250 mg/kg prednisolone 1 day before inoculation; On the day of infection with Candida albicans, mice were first anesthetized by inhalation of ether, a cotton ball dipped with 90% phenol solution was placed in a centrifuge tube with a 2 mm diameter hole at the bottom so as to burn the tongue of the mice for 50 s, and then the oral cavity of the mice was washed with normal saline; After an interval of 4 h, the cotton swab is immersed in the freshly cultured suspension of Candida albicans and directly applied to the whole oral cavity (including buccal mucosa, tongue, soft palate and other mucosal surfaces of oral cavity) of mice for 1 min; The blank control group was also treated with prednisolone and tetracycline, but the oral cavity of mice was not treated with phenol, and the oral cavity was smeared with sterile PBS. After modeling for 24 h, the oral mucosa (tongue, jaw, etc.) of mice had ulcer lesions, indicating successful modeling.

Administration after modeling of oral ulcer in mice: ulcerated mice for subsequent experiments were confimed; After the mice were anesthetized with ether, 100 µL of the gel sample was smeared onto the ulcer and its periphery using a syringe with a needle removed. The test was conducted at high doses and low doses, once daily for 8 consecutive days. Among them, the positive control group was given soft gel containing KGF-1 and the aqueous extract of the original prescription of "Gancao Xiexin decoction"; The blank control group and negative control group were given soft gel without traditional Chinese medicine; The experimental group was given the soft gel containing the aqueous extract of "Gancao Xiexin decoction" modified prescription.

### (3) Observation on body weight and ulcer healing

Mice were weighed periodically each day and oral mucosal ulcers were observed, and ulcer healing was recorded by photographing. After the complete healing of oral ulcers in one group of mice, the mice were sacrificed to extract serum, and the oral mucosal repair in each group was evaluated.

Ulcer score: after the mice in each group were anesthetized together, the tongue of the mice was pulled out of the oral cavity and photographed, and the white spots on the tongue surface of the mice were scored according to the scoring standard by single-blind observation method. The ulcer scoring criteria were as follows: 0 represents normal; 1 represents a small number of thin white spots or red and swollen, covering about 20% of the tongue surface area; 2 represents thin white spots or red and swollen, covering about 20-50% of tongue surface area; 3 represents thin white spots or red and swollen, covering 50-90% of tongue surface area; 4 represents thick pseudomembranous white spots or ulcerated edema of the entire tongue, covering> 90% of the tongue surface area. Main observation indicators include ulcer area, degree of indentation, presence or absence of pseudomembranes, degree of surrounding red and swollen.

### (4) Immune-related cytokine assay

Each group of mice was assayed in two batches of blood samples. The first sampling was performed on day 5 after 4 doses and the second on day 9 after 8 doses. The changes of immune-related cytokines at the time of the most serious development of oral ulcer and at the time of ulcer close to healing were investigated.

Specifically, the mice were anesthetized with ether and eyeballs were removed for blood collection, and the mice in each group were killed by cervical dislocation; the whole blood was collected into a centrifuge tube, allowed to stand at room temperature for 2 h, low-temperature centrifugation (15 min at 1000 g) was performed, the serum was separated and cryopreserved at -80°C for later use; the levels of IL-6, IL-1α, IL-10 and IL-35 in peripheral blood serum of mice were determined by ELISA method.

### 3. Experimental results

### (1) Evaluation of repair of experimental animal oral ulcer model

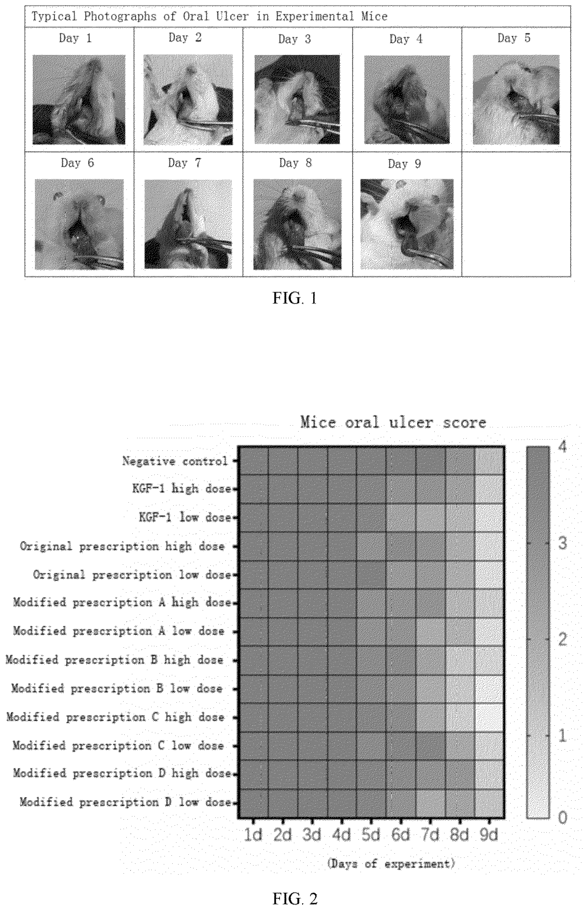

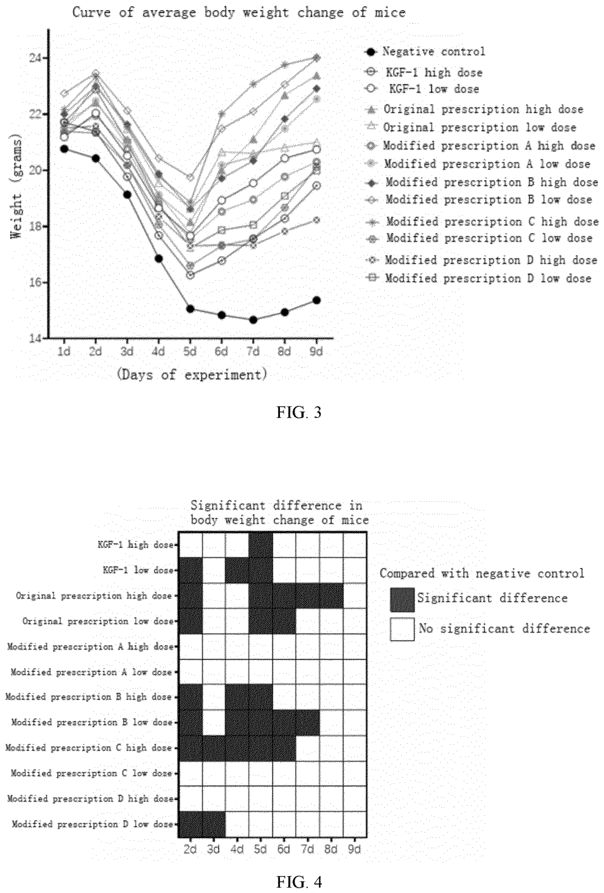

The healing process of typical oral ulcers in mice is shown in FIG. 1, which is used as a representative to illustrate the healing of oral ulcers in mice of each group. In the early stage of oral ulcer, the dorsal tongue of mice was ulcerated, red and swollen, and covered with white yellowing pseudomembrane. Combined with the data on the body weight of mice in FIG. 3, oral ulcers hindered the normal eating of mice, and the food intake and water intake of mice decreased significantly; over time, oral ulcers showed signs of improvement, but the degree of improvement was different in each group. After 5-6 days of administration, due to the effect of the sample, the oral ulcer of the mice in the positive control KGF-1 group and each modified prescription experimental group accelerated to improve, the area of tongue ulcer decreased, and their body weight began to recover; on Day 9, the ulcer on the dorsal tongue of some individuals disappeared, and the dorsal tongue returned pink as a whole, leaving only light white marks.

FIG. 2 is a heat map of the scores for oral ulcers in mice, corresponding to the tables of scores shown in Table 2, with lower scores indicating better recovery. Since there is only a slight difference in the effects of the formulations with different parts by weight in the same modified prescription, and the difference is not significant. The formulation with the relatively best effect in each modified prescription, i.e. the data corresponding to the formulation of the group (1), is used as a representative for comparison with other modified prescriptions and controls. It can be seen from FIG. 2 and Table 2 that in the mice in the negative control group, the ulcer began to improve on Day 8, but the healing degree was poor; in the other groups, KGF-1 low-dose group, original prescription high- and low-dose groups, modified prescription B low-dose group and modified prescription C high-dose group, the overall ulcer healing degree was better at Day 9 of observation; among them, the modified prescription B low-dose group and the modified prescription C high-dose group had the best healing condition.

**Table 2 Corresponding score table of mice oral ulcer score heat map**

| Group | 1d | 2d | 3d | 4d | 5d | 6d | 7d | 8d | 9d |
|---|---|---|---|---|---|---|---|---|---|
| Negative control | 4.0 | 4.0 | 4.0 | 4.0 | 4.0 | 4.0 | 4.0 | 3.0 | 1.5 |
| KGF-1 high dose | 4.0 | 4.0 | 4.0 | 4.0 | 3.7 | 3.0 | 3.0 | 2.4 | 1.0 |
| KGF-1 low dose | 4.0 | 4.0 | 4.0 | 4.0 | 3.7 | 2.3 | 2.0 | 1.7 | 0.6 |
| Original prescription low dose | 4.0 | 4.0 | 4.0 | 4.0 | 3.0 | 3.3 | 3.0 | 2.0 | 0.7 |
| Original prescription high dose | 4.0 | 4.0 | 4.0 | 4.0 | 4.0 | 2.7 | 2.7 | 2.0 | 0.6 |
| Modified prescription A low dose | 4.0 | 4.0 | 4.0 | 4.0 | 3.0 | 3.0 | 3.0 | 1.7 | 1.0 |
| Modified prescription A high dose | 4.0 | 4.0 | 4.0 | 4.0 | 3.0 | 3.0 | 2.0 | 1.9 | 0.6 |
| Modified prescription B low dose | 4.0 | 4.0 | 4.0 | 4.0 | 3.3 | 3.3 | 2.3 | 1.2 | 0.8 |
| Modified prescription B high dose | 4.0 | 4.0 | 4.0 | 4.0 | 3.3 | 3.0 | 2.0 | 1.1 | 0.3 |
| Modified prescription C low dose | 4.0 | 4.0 | 4.0 | 4.0 | 3.7 | 3.3 | 2.0 | 1.0 | 0.1 |
| Modified prescription C high dose | 4.0 | 4.0 | 4.0 | 4.0 | 3.3 | 3.7 | 3.7 | 2.1 | 0.9 |
| Modified prescription D low dose | 4.0 | 4.0 | 4.0 | 4.0 | 3.7 | 3.3 | 3.0 | 2.9 | 1.0 |
| Modified prescription D high dose | 4.0 | 4.0 | 4.0 | 4.0 | 3.7 | 3.0 | 2.0 | 2.3 | 1.3 |

### (2) Body weight change of experimental animal oral ulcer model

The curve of average body weight change of mice is shown in FIG. 3. As can be seen from FIG. 3, the body weight of each group of mice gradually decreased in the first several days, reached the minimum by Day 5, and then gradually recovered to increase, but the body weight of the mice in the negative control group recovered the slowest. In both KGF-1 groups, although weight was also recovering, the rate of increase was low. Among the mice in each group, the modified prescription B low-dose group, the modified prescription C high-dose group, and the original prescription high-dose group had the best weight recovery.

The changes in body weight of mice directly reflect the food intake and water intake of animals, and directly correspond to the status of oral ulcers and body state of animals. In order to further analyze the difference in the weight recovery of each group of mice, according to the present invention, based on the daily weight data of each mouse, the weight gain and loss values of each group of mice given samples from the previous day were analyzed and compared with the weight gain and loss values of the negative control group without administration, and the statistical results of the significance of the difference in the average body weight change of mice are shown in FIG. 4. It can be seen from FIG. 4 that number of the days of significant difference were up to 5 and more in the original prescription high-dose group, the modified prescription B low-dose group and the modified prescription C high-dose group.

The present invention subsequently selected the blank control group, the negative control group, the original prescription high-dose group, the modified prescription B low-dose group, the modified prescription C high-dose group, and the KGF-1 low-dose group, and detected the changes in the contents of immune-related cytokines in the serum of the mice.

### (3) Changes of immune-related cytokines in the blood of mice

### (1) Interleukin-6 (IL-6)

IL-6 is mainly secreted by Th2 cells and is a pleiotropic cytokine involved in the mediation of inflammatory response and immune response. IL-6, as a pro-inflammatory cytokine, regulates the early immune response of B lymphocytes, macrophages, and NK cells and is elevated in a variety of autoimmune and inflammatory diseases. IL-6 has also been reported to induce fibroblasts to produce collagen.

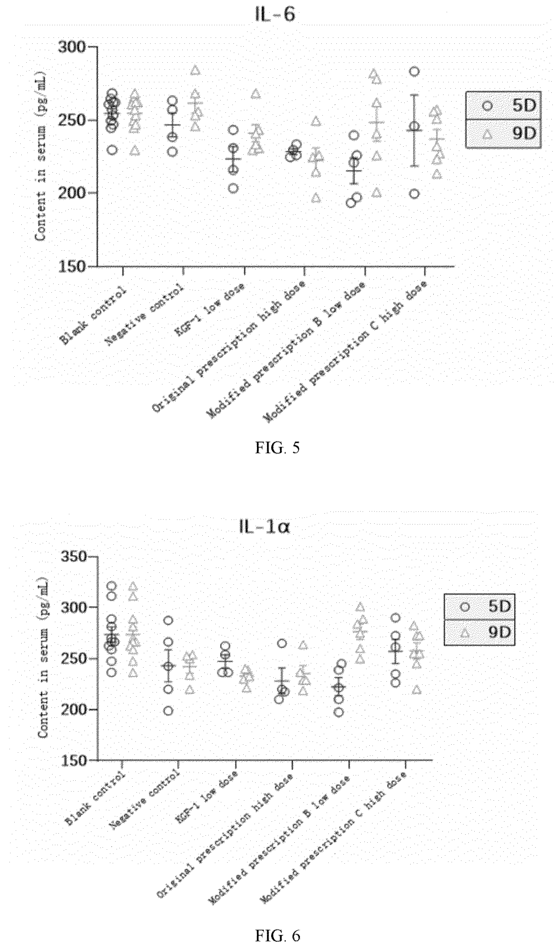

The results of the change in the content of IL-6 in the blood of mice are shown in FIG. 5, it can be seen from FIG. 5 that the IL-6 content in the serum of each experimental group was decreased in varying degrees compared with the blank control group on Day 5. Combined with the healing of oral ulcer in mice, the modified prescription of Gancao Xiexin decoction of the present invention can inhibit IL-6, thereby reducing the inflammatory response. However, as the administration was continued, the IL-6 content of each group was greatly increased by Day 9, with the increase of the KGF-1 group and modified prescription B low-dose group being greater, indicating that it may show the effect of promoting collagen synthesis.

### (2) Interleukin-1α (IL-1α)

IL-1α is expressed in a variety of cells, including epithelial cells, endothelial cells, and stromal cells, and can be up-regulated by a variety of stimuli in hematopoietic and non-hematopoietic cells, and is a common inflammatory factor.

The results of the change of IL-1α content in the blood of mice are shown in FIG. 6. It can be seen from FIG. 6 that the IL-1α content in the blood of normal mice was the highest, while the IL-1α content in the blood of all the mice with oral ulcer model showed a decrease. On Day 9, the modified prescription B low-dose group showed a large increase, which was close to normal; It is shown that the modified prescription of Gancao Xiexin decoction of the present invention can inhibit the inflammatory response by reducing the level of IL-1α, but does not continuously reduce the level of IL-1α.

### (3) Interleukin-10 (IL-10)

IL-10 and IL-6 belong to the Th2 cell secretory factor, both endogenous and exogenous IL-10 can strongly inhibit the synthesis of pro-inflammatory cytokines such as IL-1, IL-6, IL-8, and TNF-α at the transcriptional level, thus playing an anti-inflammatory role.

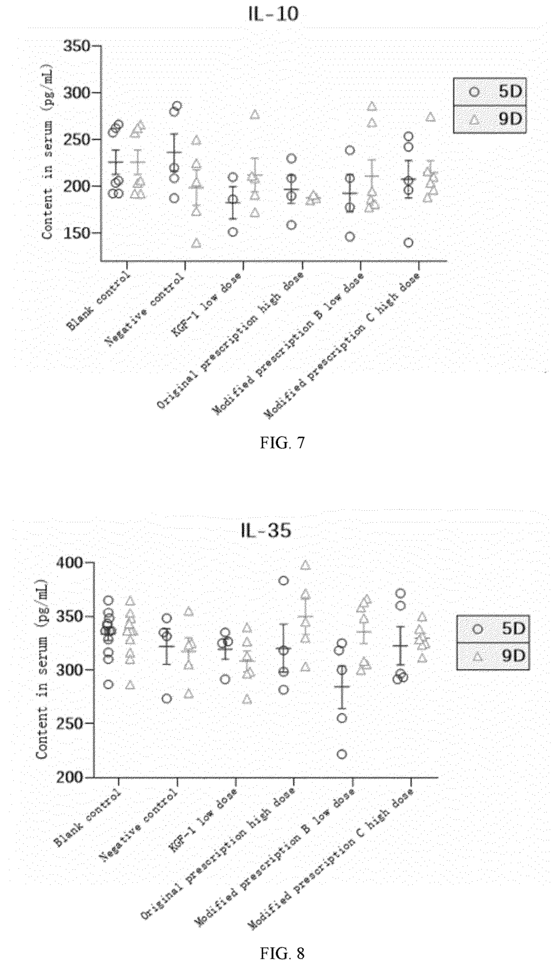

The results of changes in the IL-10 content in the blood of mice are shown in FIG. 7. It can be seen from FIG. 7 that except for the negative control group, the IL-10 content in the serum of each group of mice showed a decrease on day 5, but when the ulcer began to heal at a later stage, the IL-10 content was increased to inhibit excessive inflammatory response and promote ulcer wound healing; KGF-1 low-dose group and modified prescription B low-dose group the development of ulcer; It was shown that the modified prescription of Gancao Xiexin decoction of the present invention can inhibit inflammatory reaction by increasing the content of IL-10.

### (4) Interleukin-35 (IL-35)

IL-35 is mainly derived from regulatory T cells and is highly expressed in bone marrow, thymus, liver, and blood. IL-35 has been found to play a role in a variety of immune-related diseases, such as allergic airway disease, inflammatory bowel disease, collagen-induced arthritis, etc. Its main biological effects are the inhibition of proliferation and differentiation of T lymphocytes, inhibition of Th17 cells, inhibition of expression of pro-inflammatory cytokines, and maintenance of immune tolerance through immunosuppression, i.e. IL-35 is an immunosuppressive cytokine.

The results of changes in IL-35 content in mouse blood are shown in FIG. 8. It can be seen from FIG. 8 that the changes of IL-35 in the modified prescription B low-dose group are similar to the changes of IL-10, both decreasing at first and then increasing, while the other groups are not much different from the normal group; it showed that the low dose of modified prescription B adjusted the effect of immunosuppression, which was beneficial to ulcer healing.

The above results indicate that the modified prescription of "Gancao Xiexin decoction" of the present invention can promote the healing of oral ulcers, regulate immune response, and inhibit inflammatory response. Among them, from the healing rate of oral ulcers, weight recovery, and the changes of immune-related cytokines in serum of experimental animals, the effects of the modified prescription B and C of "Gancao Xiexin decoction" in experimental dosage were better than the original prescription, and the modified prescription C of "Gancao Xiexin decoction" had the best performance.

The embodiments described above are preferred embodiments of the present invention, but the embodiments of the present invention are not limited to the embodiments described above, and any other changes, modifications, substitutions, combinations, and simplifications made without departing from the spirit and principles of the present invention are to be construed as equivalent substitutions within the scope of the present invention.

## Claims

1. A modified prescription of Gancao Xiexin decoction, **characterized by** comprising, in parts by weight, 2-5 parts of *Coptidis Rhizoma,* 6-12 parts of *Scutellariae Radix,* 10-15 parts of *Glycyrrhizae Radix et Rhizoma,* 6-12 parts of *Zingiberis Rhizoma,* 5-12 parts of *Jujubαe Fructus,* 10-15 parts of *Citri Reticulatae Pericarpium,* and 10-15 parts of *Bolbostemmatis Rhizoma.*

2. The modified prescription of Gancao Xiexin decoction of claim 1, **characterized by** further comprising, in parts by weight, 1-5 parts of *Pogostemonis Herba* and 1-5 parts of *Eupatorii Herba.*

3. The modified prescription of Gancao Xiexin decoction of claim 1, **characterized by** further comprising, in parts by weight, 1-6 parts of *Platycodonis Radix.*

4. The modified prescription of Gancao Xiexin decoction of claim 3, **characterized by** further comprising, in parts by weight, 10-20 parts of *Scrophulariae Radix* and 10-20 parts of *Ophiopogonis Radix.*

5. Use of the modified prescription of Gancao Xiexin decoction of any one of claims 1 to 4 or an extract thereof in the preparation of a product for treating oral ulcer.

6. A product for treating oral ulcer, **characterized in that** the product comprises the modified prescription of Gancao Xiexin decoction of any one of claims 1 to 4 or the extract thereof.

7. The product of claim 6, **characterized in that** the extract is an aqueous extract of the modified prescription of Gancao Xiexin decoction of any one of claims 1 to 4.

8. The product of claim 7, **characterized in that** the preparation of the aqueous extract of the modified prescription of Gancao Xiexin decoction comprises the following steps of:
S1, taking medicinal materials according to the modified prescription, removing insects or metamorphic parts thereof, crushing the medicinal materials and sieving to obtain crude medicinal powder;
S2, adding pure water to the crude medicinal powder obtained in step S1 in a ratio of solid to liquid of 1:(10-40), heating, refluxing to extract, and obtaining an extract liquid; and
S3, centrifuging the extract liquid obtained in step S2, and concentrating a supernatant in vacuo to obtain the aqueous extract of the modified prescription of Gancao Xiexin decoction.

9. A soft gel for treating oral ulcer, **characterized by** comprising the aqueous extract of the modified prescription of Gancao Xiexin decoction of any one of claims 1 to 4.

10. A method for preparing the soft gel of claim 9, **characterized by** after diluting the aqueous extract of the modified prescription of Gancao Xiexin decoction with water, adding sodium carboxymethyl cellulose, glycerol, and sucralose for heating and dissolving, and cooling to obtain the soft gel.
